# FASCICULE DE BREVET EUROPEEN

(11) **EP 0 646 412 B1**
(45) Date de publication et mention de la délivrance du brevet: **19.05.1999**
(21) Numéro de dépôt: 94402019.7
(22) Date de dépôt: 09.09.1994
(51) Int. Cl.: B01J 31/24, C07C 2/36

(54) **Composition catalytique et procédé pour la dimérisation des oléfines**
Katalytische Zusammensetzung und Verfahren zum Dimerisieren von Olefinen
Catalyst composition and process for the dimerisation of olefins

(30) Priorité: 22.09.1993 FR 9311381
(43) Date de publication de la demande: 05.04.1995
(73) Titulaire: INSTITUT FRANCAIS DU PETROLE, 92502 Rueil-Malmaison (FR)
(72) Inventeur: Chauvin, Yves, F-78230 Le Pecq (FR); Einloft, Sandra, Rio Grande do Sul CPE 900040 Bresil (BR); Olivier, Hélène, F-92500 Rueil Malmaison (FR)

(56) Documents cités:
- EP-A- 0 448 445
- FR-A- 2 220 493
- US-A- 510 840

## Description

La présente invention concerne une composition catalytique et un procédé utilisant cette composition pour la dimérisation, la codimérisation et l'oligomérisation des oléfines et en particulier du propylène, la composition résultant de la dissolution d'un composé du nickel mélangé ou complexé avec une phosphine, dans le mélange liquide, a caractère ionique, d'halogénure d'ammonium quaternaire ou/et d'halogénure de phosphonium quaternaire, d'halogénure d'aluminium, d'un hydrocarbure aromatique et optionnellement d'un composé d'alcoyle aluminium.

Dans le brevet français 2611700 est décrite l'utilisation de liquides a caractère ionique formés d'halogénures d'aluminium et d'halogénures d'ammonium quaternaires comme solvants de complexes organométalliques du nickel pour la catalyse de dimérisation des oléfines. L'utilisation de tels milieux non miscibles avec les hydrocarbures aliphatiques, en particulier avec les produits issus de la dimérisation des oléfines permet une meilleure utilisation des catalyseurs homogènes. Dans le brevet U.S. 5.104.840 est décrite une composition liquide à caractère ionique résultant de la mise en contact d'halogénures d'ammonium quaternaires et/ou d'halogénures de phosphonium quaternaires avec des dihalogénures d'alcoyle aluminium et éventuellement en outre un trihalogénure d'aluminium. Ce même brevet décrit l'utilisation de ces milieux comme solvants de complexes de métaux de transition, notamment des complexes du nickel ne contenant pas de liaison nickel-carbone, qui sont transformés en catalyseurs d'oligomérisation des oléfines. Dans la suite ces milieux seront appelés "sels fondus" parce que liquides à température modérée.

Au cours de ces travaux il a été montré que les catalyseurs de nickel les plus actifs et les plus stables sont obtenus dans des "sels fondus" constitués d'un équivalent molaire d'halogénure d'ammonium et/ou d'halogénure de phosphonium avec un équivalent et plus de trihalogénure d'aluminium, et optionnellement une quantité quelconque de dihalogénure d'alcoyle aluminium. Cette formulation s'est révélée particulièrement intéressante parce que les complexes du nickel qui y sont dissous présentent une activité catalytique élevée et constante dans le temps.

Cependant il s'est révélé que dans de telles conditions "l'effet phosphine" tel que décrit par G. Wilke et al dans Ind. Eng Chem., 1970, 62, n°12, p34, et dans le brevet G.B. 1.058.680, et qui traduit l'influence des substituants portés par l'atome de phosphore sur le mode d'enchaînement des molécules de propylène lors de la dimérisation catalytique par le nickel, disparaissait rapidement au cours du temps. Ce phénomène inexpliqué a des conséquences néfastes puisqu'il ne permet pas d'obtenir les sélectivités recherchées.
Il a maintenant été trouvé que l'addition d'un hydrocarbure aromatique à un "sel fondu" permet de palier ce défaut et conduit a des catalyseurs dont l'activité est élevée et stable et dont la sélectivité en isomères les plus ramifiés est importante.

Plus précisément un objet de l'invention est une composition catalytique comprenant au moins un composé du nickel mélangé ou complexé avec au moins une phosphine tertiaire, dissous au moins en partie dans un milieu non aqueux à caractère ionique résultant de la mise en contact d'au moins un halogénure d'aluminium (B) avec au moins un halogénure d'ammonium quaternaire et/ou au moins un halogénure de phosphonium quaternaire (A) et avec au moins un hydrocarbure aromatique (C).
Un autre objet de l'invention est un procédé pour la dimérisation, la codimérisation ou l'oligomérisation d'au moins une oléfine, procédé dans lequel l'oléfine est mise au contact d'au moins un composé du nickel mélangé ou complexé avec au moins une phosphine tertiaire, ledit composé étant dissous au moins en partie dans milieu non-aqueux à caractère ionique, procédé caractérisé en ce que ledit milieu résulte de la mise en contact d'au moins un halogénure d'aluminium avec au moins un halogénure d'ammonium quaternaire et/ou de phosphonium quaternaire et avec au moins un hydrocarbure aromatique

Le milieu de type "sels fondus" est donc constitué:
a) d'halogénures, plus particulièrement chlorures et/ou bromures, d'ammonium quaternaires et/ou phosphonium quaternaires (noté produit A);
b) d'halogénure d'aluminium et de préférence le chlorure, le bromure (noté produit B);
c) d'hydrocarbure aromatique simple, condensé ou substitué (noté produit C);
d) optionnellement d'un dérivé organique de l'aluminium (noté produit D).

Les halogénures d'ammonium quaternaires et les halogénures de phosphonium quaternaires utilisables dans le cadre de l'invention répondent de préférence aux formules générales NR^{l}R²R³R⁴X et PR^{l}R²R³R⁴X, où X représente Cl ou Br, R^{l}, R², R³ et R⁴, identiques ou différents, représentant chacun l'hydrogène, un groupement alkyle, aliphatique (saturé ou insaturé) ou aromatique, comprenant 1 a 12 atomes de carbone. Les halogénures d'ammonium et/ou de phosphonium quaternaires peuvent également être dérivés d'hétérocycles comportant 1, 2 ou 3 atomes d'azote et/ou de phosphore. A titre d'exemples on peut citer le chlorure de tétrabutylphosphonium, le chlorure de N-butylpyridinium, le bromure d'éthyl pyridinium, le chlorure de butyl-3 méthyl- 1 imidazolium, le chlorure de diéthylpyrazolium, le chlorhydrate de pyridinium, le chlorure de triméthylphényl ammonium, .

Les hydrocarbures aromatiques selon l'invention sont le benzène et ses substitués de formule générale C₆H_{X}R_{6-X}, R étant un radical alkyl, cycloalkyl, aryl, alkylaryl tel que C₆H₅CH₂-, et x prenant les valeurs de 1 à 5 ; le naphtalène et ses substitués de formule générale C₁₀H_{X}R₈₋ₓ, R étant défini comme ci-dessus et x étant compris entre 0 et 7; I'anthracène et ses dérivés de formule générale C₁₄HxR₁₀₋ₓ où R tel que défini précédemment, x égal de 0 à 9 inclus..

Ils peuvent être utilisés seuls ou en mélange. A titre d'exemples on peut citer le benzène, le toluène, les xylènes, le durène et l'isodurène, le pentaméthylbenzène, l'hexaméthylbenzène, l'a-méthylnaphtalène, le diméthyl-2,6 anthracène.

Les dérivés organiques de l'aluminium selon l'invention ont pour formule générale AlR_{X}X₃₋ₓ dans laquelle R est un radical alkyl, linéaire ou ramifié, comportant 2 à 8 atomes de carbone, X étant le chlore ou le brome et x ayant une valeur égale à 1, 2 ou 3. A titre d'exemples on peut utiliser le dichloroéthylaluminium, le sesquichlorure d'éthylaluminium, le sesquichlorure d'isobutylaluminium, le dichloroisobutylaluminium et le chlorodiéthylaluminium.

Les composants des "sels fondus" tels que definis ci-dessus sont mis en oeuvre dans des rapports molaires A:B compris entre 1:0,5 et 1:3, de préférence entre 1:1 et 1:2; B:C compris entre 1:1 et 1:100, de préférence entre 1:1 et 1:10 et B:D compris entre 1:0 et 1: 10 de préférence entre 1:0,01 et 1:5. Il est néanmoins nécessaire que les composants et leurs proportions soient tels que le mélange soit liquide à la température à laquelle se fait l'introduction du composé du nickel et la phosphine, bien que la réaction catalytique de dimérisation puisse se faire à une température inférieure ou supérieure à la température de fusion de la composition catalytique. Si l'hydrocarbure aromatique se partage entre la phase polaire et la phase hydrocarbonée que constitue les dimères et les oligomères, il est nécessaire d'ajouter de façon continue de cet hydrocarbure aromatique en sorte que sa concentration dans la phase polaire reste dans la fourchette donnée ci-dessus.

Les composés entrant dans la composition selon l'invention, peuvent être mélangés dans un ordre quelconque. Le mélange peut se faire par une simple mise en contact suivie d'une agitation jusqu'à formation d'un liquide homogène. Ce mélange peut être fait en dehors du réacteur de dimérisation ou de préférence dans ce réacteur.

Les composés du nickel selon l'invention sont le chlorure, le bromure, le sulfate, les carboxylates, par exemple l'éthyl-2 hexanoate, les phénates, l'acétylacétonate, en mélange avec une phosphine tertiaire, ou leurs complexes avec une phosphine tertiaire. On peut également utiliser les complexes organométalliques du nickel contenant ou non des phosphines

Les phosphines selon l'invention répondent aux formules générales PR^{l}R²R³ et R^{l}R²P-R'-PR^{l}R² dans lesquels R^{l},R² et R³ identiques ou différents, sont des radicaux alkyl, cycloalkyl, aryl ou aralkyl comportant de 1 a 10 atomes de carbone, et R' un reste bivalent aliphatique de 1 a 6 atomes de carbone.

A titre d'exemples on peut citer :
la triisopropylphosphine,
la tricyclohexylphosphine,
la tribenzylphosphine,
la dicyclohexylphenylphosphine,
la tétra cyclohexylmethylene-diphosphine
la diisopropyltertiobutylphosphine.

A titre d'exemples de composés du nickel utilisables selon l'invention on peut citer les complexes NiCl₂,2P(isopropyl)₃, NiCl₂,2P(cyclohexyl)₃, NiCl₂,2pyridine en mélange avec un équivalent de triisopropylphosphine, le chlorure de nickel en mélange avec un équivalent de triisopropylphosphine, I'acétate de nickel en mélange avec un équivalent de tricyclohexylphosphine, le chlorure de pallylnickeltriisopropylphosphine.

Les oléfines susceptibles d'être dimérisées ou oligomérisées par les compositions catalytiques selon l'invention sont l'éthylène, le propylène, les n-butènes et les n-pentènes, seules ou en mélange, pures ou diluées par un alcane, telles qu'on les trouve dans des "coupes" issues des procédés de raffinage du pétrole, comme le craquage catalytique ou le craquage a la vapeur.

La réaction catalytique de dimérisation des oléfines peut être conduite en système fermé, en système semi-ouvert ou en continu avec un ou plusieurs étages de réaction. Une vigoureuse agitation doit assurer un bon contact entre le ou les réactifs et la composition catalytique. La température de réaction peut être comprise entre -40 et +70 °C, de préférence entre -20 et +50 °C. On peut opérer au dessus ou en dessous de la température de fusion de la composition catalytique, I'état solide dispersé n'étant pas une limitation au bon déroulement de la réaction. La chaleur engendrée par la réaction peut être éliminée par tous les moyens connus de l'homme de l'art.

La pression peut être comprise entre 0,1 MPa et 20 Mpa, de préférence entre la pression atmosphérique et 5 MPa. Les produits de la réaction et le ou les réactifs qui n'ont pas réagi sont séparés du système catalytique par simple décantation puis fractionnés.

Les exemples suivants illustrent l'invention sans en limiter la portée:

### EXEMPLE 1

### Préparation du solvant ionique.

On a mélangé à température ambiante 17,5g (0,1 mole) de chlorure de butylméthyl imidazolium, 16,3g (0,122 mole) de chlorure d'aluminium sublimé, 0,26 g (0,002 mole) de dichloroéthylaluminium et 4,02g (0,03 mole) d'isodurène. On a obtenu ainsi un liquide jaune claire.

### Dimérisation du propylène.

Un réacteur en verre de 100 ml muni d'une sonde de mesure de température, d'un barreau aimanté pour assurer une bonne agitation et d'une double enveloppe permettant la circulation d'un liquide de réfrigération, a été purgé d'air et d'humidité, et maintenu à la pression atmosphérique de propylène à 99% de pureté. On y a introduit 45 mg (0,1 mmole) du complexe NiCl₂,2P(iPr)₃ puis on a abaissé la température a -15 °C et injecté à l'aide d'une seringue 3,5 ml de la composition liquide ci-dessus préparée et 7 ml d'heptane. On a mis l'agitation en route et on a observé immédiatement une absorption de propylène. Quand le réacteur a été aux trois quarts plein de liquide, on a arrêté l'agitation, on a laissé se décanter le "sel fondu" et on a soutiré la plus grande partie de la phase hydrocarbonée. On a recommencé l'opération sept fois. A ce moment on avait introduit au total 430g de propylène. L'analyse des différentes fractions à montré qu'elles étaient composées à 85 % de dimères, à 12 % de trimères et à 3 % de tétramères. La composition des dimères qui était pratiquement identique dans toutes les fractions comportait 81 % de diméthyl-2,3 butènes, 2 % de n-hexènes et 17 % de méthyl-2 pentènes. Cette teneur en diméthylbutènes est bien supérieure à celle qui avait été décrite par G. Wilke.

### EXEMPLE 1' (comparatif)

### Préparation du solvant ionique.

On a préparé un solvant ionique dans des conditions identiques à celui de l'exemple précédent à ceci près qu'il n'a pas été ajouté d'hydrocarbure aromatique. Le liquide était dans ce cas pratiquement incolore.

### Dimérisation du propylène.

On a opéré comme dans l'exemple précédent. La première fraction hydrocarbonée s'est révélée être composée de 83 % de dimères, 14 % de trimères et 3 % de tétramères les dimères contenaient 83 % de diméthyl-2,3 butènes, 2 % de n-hexènes et 15 % de méthyl-2 pentènes. Les dimères de la septième fraction, qui représentaient toujours 85 % des produits, ne contenaient plus que 11 % de diméthyl-2,3 butènes à côté de 16 % de n-hexènes et de 63 % de méthyl-2 pentènes. La composition de la dernière fraction est particulièrement basse en diméthylbutènes.

### EXEMPLE 2

### Dimérisation du propylène

On a opéré comme dans l'exemple 1 à ceci près qu'au lieu de 45 mg du complexe NiCl₂,2P(iPr)₃ on a introduit 69 mg (0,1 mmole) du complexe NiCl₂,2P(cyclohexyl)₃. On a effectué trois soutirages, ce qui correspondait à 210 g de propylène introduit. Les trois fractions étaient constituées à 78% de dimères, à 18% de trimères et à 4% de tétramères. Les dimères contenaient
84% de diméthyl-2,3 butènes, de 1% de n-héxènes et de 15% de méthyl-2 pentènes. Les dimères étaient particulièrement riches en diméthylbutènes.

### EXEMPLE 3

### Préparation du solvant ionique

On a opéré comme dans l'exemple 1 à ceci près que l'isodurène a été remplacé par 4,26 g d'a-méthylnaphtalène.

### Dimérisation du propylène

On a opéré comme dans l'exemple 1 à ceci près qu'on a utilisé le "sel fondu " préparé à cet effet, et qu'on a introduit 50 mg (0.12 mmole) du complexe NiCl₂,2PiPr₃. On a effectué trois soutirages. La première fraction était composée à 78% de dimères qui contenaient 84% de diméthyl-2,3 butènes. La dernière fraction était composée à 88% de dimères qui contenaient 65% de diméthyl-2,3 butènes.

### EXEMPLE 4

### Préparation du solvant ionique

On a opéré comme dans l'exemple 1 à ceci près que l'isodurène a été remplacé par 4,4 g de pentaméthylbenzène

### Dimérisation du propylène

On a opéré comme dans l'exemple 1 à ceci près qu'on a utilisé le "sel fondu " préparé à cet effet, et qu'on a introduit 50 mg (012 mmole) du complexe NiCl₂,2PiPr₃. On a effectué six soutirages correspondant à 370 g de propylène introduit. La première fraction était composée à 79% de dimères qui contenaient 83% de diméthyl-2,3 butènes. La dernière fraction était composée à 84% de dimères qui contenaient 75% de diméthyl-2,3 butènes.

### EXEMPLE 5

Cet exemple illustre le cas pour lequel l'hydrocarbure aromatique, ici le toluène, se partage entre la phase polaire et la phase constituée par les oligomères. Il en est alors ajouté après chaque soutirage.

### Préparation du solvant ionique

On a opéré comme dans l'exemple 1 à ceci près que l'isodurène a été remplacé par 2,46 g de toluène.

### Dimérisation du propylène

On a opéré comme dans l'exemple 1 à ceci près qu'on a utilisé le "sel fondu " préparé à cet effet, et qu'on a introduit 50 mg (0,12 mmole) du complexe NiCl₂,2PiPr₃. On a effectué six soutirages correspondant à 370 g de propylène introduit. Après chaque soutirage on a ajouté 0,2 mL de toluène .La première fraction était composée à 78% de dimères qui contenaient 83% de diméthyl-2,3 butènes. La dernière fraction était composée à 78% de dimères qui contenaient 83% de diméthyl-2,3 butènes. Dans un essai identique dans lequel on n'avait pas ajouté de toluène après chaque soutirage la dernière fraction ne contenait plus que 10% de diméthylbutènes.
Il est bien entendu que dans un procédé fonctionnant en continu, le toluène serait ajouté en continu ou périodiquement dans le mélange agité de la composition catalytique et des produits de réaction.

## Revendications

1. Composition catalytique comportant au moins un composé du nickel mélangé ou complexé avec au moins une phosphine tertiaire, dissous au moins en partie dans un milieu non-aqueux à caractère ionique résultant de la mise en contact d'au moins un halogénure d'aluminium (B), avec au moins un halogénure d'ammonium quaternaire ou/et d'au moins un halogénure de phosphonium quaternaire (A), et avec au moins un hydrocarbure aromatique (C) .

2. Composition catalytique selon la revendication 1 dans laquelle l'halogénure d'ammonium quaternaire est choisi dans le groupe contitué par le chlorure de N-butylpyridinium, le bromure d'éthylpyridinium, le chlorure de butyl-3méthyl-1 imidazolium, le chlorure de diéthylpyrazolium et le chlorure de N-butylpyridinium.

3. Composition catalytique selon l'une des revendications précédentes dans laquelle l'halogénure de phosphonium quaternaire est le chlorure de tétrabutylphosphonium.

4. Composition catalytique selon l'une des revendications précédentes dans laquelle l'halogénure d'aluminium est le chlorure d'aluminium.

5. Composition catalytique selon l'une des revendications précédentes dans laquelle l'hydrocarbure aromatique est choisi dans le groupe formé par le benzène, les substitués du benzène de formule générale C₆HₓR₆₋ₓ où R est un radical alkyl, cycloalkyl, aryl, alkylaryl et où x égal de 1 à 5 inclus, le naphtalène, les substitués du naphtalène de formule générale C₁₀HₓR₈₋ₓ avec R tel que défini précédemment et x égal de 0 à 7 inclus, I'anthracène, les substitués de l'anthracène de formule générale C₁₄HₓR₁₀₋ₓ avec R tel que défini précédemment et x égal de 0 à 9 inclus.

6. Composition catalytique selon la revendication 5, dans laquelle l'hydrocarbure aromatique est choisi dans le groupe formé par le toluène, les xylènes, le durène, l'isodurène, le pentaméthylbenzène, l'a-méthylnaphtalène, le diméthyl-2,6 anthracène.

7. Composition catalytique selon l'une des revendications précédentes dans laquelle le milieu non-aqueux contient également un dérivé organique de l'aluminium (D) de formule générale AlRₓX₃₋ₓ où R est un radical alkyl, linéaire ou ramifié, comportant de 2 à 8 atomes de carbone, X est le chlore ou le brome et x est égal à 1, 2 ou 3.

8. Composition catalytique selon la revendication 7 dans laquelle le dérivé organique de l'aluminium est choisi dans le groupe formé par le dichloroéthylaluminium, le dichloroisobutylaluminium, le chlorodiéthylaluminium, le sesquichlorure d'éthylaluminium, le sesquichlorure de di-isobutylaluminium.

9. Composition catalytique selon l'une des revendications précédentes, dans laquelle le rapport molaire A : B est compris entre l:0,5 et 1:3, le rapport molaire B : C est compris entre 1:1 et 1:100.

10. Composition catalytique selon l'une des revendications précédentes dans laquelle le rapport molaire A : B est préférentiellement compris entre 1:1 et 1:2, le rapport molaire B : C est préférentiellement compris entre 1:1 et 1:10.

11. Composition catalytique selon l'une des revendications 7 à 10 dans laquelle le rapport molaire B : D est compris entre 1:0 et 1:10.

12. Composition catalytique selon l'une des revendications 7 à 11 dans laquelle le rapport molaire B : D est préférentiellement compris entre 1:0,01 et 1:5.

13. Composition catalytique selon l'une des revendications précédentes, dans laquelle le composé du nickel est choisi dans le groupe formé par le chlorure, le bromure, le sulfate, I'acétylacétonate, les carboxylates, les phénates de nickel.

14. Composition catalytique selon l'une des revendications précédentes, dans laquelle la phosphine est choisie dans le groupe formé par la triisopropylphosphine, la tricyclohexylphosphine, le tribenzylphosphine et la tétracyclohexylméthylènediphosphine.

15. Procédé pour la dimérisation, la codimérisation ou l'oligomérisation d'au moins une oléfine, procédé dans lequel l'oléfine est mise en contact d'au moins un composé du nickel mélangé ou complexé avec au moins une phosphine tertiaire, ledit composé étant dissous au moins en partie, dans un milieu non-aqueux à caractère ionique, procédé caractérisé en ce que ledit milieu résulte de la mise en contact d'au moins un halogénure d'aluminium avec au moins un halogénure d'ammonium quaternaire et/ou au moins un halogénure de phosphonium quaternaire et avec au moins un hydrocarbure aromatique.

16. Procédé selon la revendication 15 dans lequel la température de réaction est comprise entre -40°C et +70°C et la pression comprise entre 0,1 et 20 MPa.

17. Procédé selon l'une des revendications 15 ou 16 dans lequel la température de réaction avec l'oléfine est comprise entre -20°C et +50°C.

18. Procédé selon l'une des revendications 15 à 17 dans lequel de l'hydrocarbure aromatique est introduit pendant la réaction avec l'oléfine.

## Claims

1. A catalytic compound comprising at least one nickel compound mixed or complexed with at least one tertiary phosphine. dissolved at least partly in a non-aqueous medium of ionic type resulting from the contacting of at least one aluminium halide (B) with at least one quaternary ammonium halide and/or at least one quaternary phosphonium halide (A), and with at least one aromatic hydrocarbon (C).

2. A catalytic composition according to Claim 1 in which the quaternary ammonium halide is selected from the group constituted by N-butyl pyridinium chloride, ethylpyridinium bromide, 3-butyl chloride 1-methyl imidazolium, diethyl pyrazolium chloride and N-butylpyridinium chloride.

3. A catalytic composition according to one of the preceding claims in which the quaternary phosphonium halide is tetrabutylphosphonium chloride.

4. A catalytic composition according to one of the preceding claims in which the aluminium halide is aluminium chloride.

5. A catalytic composition according to one of the preceding claims in which the aromatic hydrocarbon is selected from the group formed by benzene, benzene substitutes of the general formula C₆HₓR₆₋ₓ where R is an alkyl, cycloalkyl, aryl, alkaryl radical and where x is equal to 1 to 5 inclusive, naphthalene, naphthalene substitutes of the general formula C₁₀HₓR₈₋ₓ with R such as defined hereinabove and x equal to 0 to 7 inclusive, anthracene, anthracene substitutes of the general formula C₁₄HₓR₁₀₋ₓ with R as defined hereinabove and x equal to 0 to 9 inclusive.

6. A catalytic composition according to Claim 5 in which the aromatic hydrocarbon is selected from the group formed by toluene, xylenes, durene, isodurene, pentamethylbenzene, a-methylnaphthalene, 2,6-dimethylanthracene.

7. A catalytic composition according to one of the preceding claims, in which the non-aqueous medium also contains an organic aluminium derivative (D) of the general formula: AlRₓX₃₋ₓ where R is a linear or branched alkyl radical comprising 2 to 8 carbon atoms, X is chlorine or bromine and x is equal to 1.2 to 3.

8. A catalytic composition according to Claim 7 in which the organic aluminium derivative is selected from the group formed by dichloroethylaluminium, dichloroisobutylaluminium, chlorodiethylaluminium, ethylaluminium sesquichloride, diisobutylaluminium sesquichloride.

9. A catalytic composition according to one of the preceding claims in which the A:B molar ratio is between 1:0.5 and 1:3, the B:C molar ratio is between 1:1 and 1:100.

10. A catalytic composition according to one of the preceding claims in which the A:B molar ratio is preferably between 1:1 and 1:2, the B:C molar ratio is preferably between 1:1 and 1:10.

11. A catalytic composition according to one of Claims 7 to 10 in which the B:D molar ratio is between 1:0 and 1:10.

12. A catalytic composition according to one of Claims 7 to 11 in which the B:D molar ratio is preferably between 1:0.01 and 1:5.

13. A catalytic composition according to one of the preceding claims in which the nickel compound is selected from the group formed by chloride, bromide, sulphate, acetylacetonate, carboxylates, nickel phenates.

14. A catalytic composition according to one of the preceding claims in which the phosphine is selected from the group formed by triisopropylphosphine, tricyclohexylphosphine, tribenzylphosphine and tetracyclohexylmethylenediphosphine.

15. A process for the dimerization, codimerization or oligomerisation of at least one olefin, in which process the olefin is contacted with at least one nickel compound mixed or complexed with at least one tertiary phosphine, said compound being dissolved at least partly in a non-aqueous medium of ionic type, the process being characterised in that said medium results from contacting at least one aluminium halide with at least one quaternary ammonium halide and/or at least one quaternary phosphonium halide and with at least one aromatic hydrocarbon.

16. A process according to Claim 15 in which the reaction temperature is between -40°C and +70°C and the pressure is between 0.1 and 20 MPa.

17. A process according to one of Claims 15 or 16 in which the reaction temperature with the olefin is between -20°C and +50°C.

18. A process according to one of Claims 15 to 17 in which aromatic hydrocarbon is introduced during the reaction with the olefin.

## Patentansprüche

1. Katalytische Zusammensetzung, umfassend wenigstens eine Verbindung des Nickels im Gemisch oder im Komplex mit wenigstens einem tertiären Phosphin, welche wenigstens zu einem Teil in einem nicht-wässrigen Medium mit ionischem Charakter gelöst ist, das aus der Kontaktierung wenigstens eines Aluminiumhalogenids (B) mit wenigstens einem quarternären Ammoniumhalogenid und/oder wenigstens einem quarternären Phosphoniumhalogenid (A) und mit Wenigstens einem aromatischen Kohlenwasserstoff (C) erhalten wird.

2. Katalytische Zusammensetzung nach Anspruch 1, bei der das quarternäre Ammoniumhalogenid aus der Gruppe ausgewählt ist, die durch N-butylpyridiumchlorid, Ethylpyridiniumbromid, 3-butyl-1-methyl-imidazoliumchlorid, Di-ethylpyrazoliumchlorid und N-butylpyridiniumchlorid gebildet ist.

3. Katalytische Zusammensetzung nach einem der vorhergehenden Ansprüche, bei der das quarternäre Phosphoniumhalogenid Tetrabutylphosphoniumchlorid ist.

4. Katalytische Zusammensetzung nach einem der vorhergehenden Ansprüche, bei der das Aluminiumhalogenid Aluminiumchlorid ist.

5. Katalytische Zusammensetzung nach einem der vorhergehenden Ansprüche, bei der der aromatische Kohlenwasserstoff gewählt ist aus der Gruppe, die gebildet ist durch Benzen, substitutierte Benzene der allgemeinen Formel C₆HₓR₆₋ₓ, wobei R ein Alkyl-, ein Cykloalkyl-, ein Aryl- oder ein Alkylaryl-Rest ist und wobei x gleich 1 bis einschließlich 5 ist, Naphtalen, substituierte Naphtalene der allgemeinen Formel C₁₀HₓR₈₋ₓ, wobei R wie vorher definiert ist und x gleich 0 bis 7 einschließlich ist, Anthracen, substituierte Anthracene der allgemeinen Formel C₁₄HₓR₁₀₋ₓ, wobei R wie vorher definiert ist und x gleich 0 bis 9 einschließlich ist.

6. Katalytische Zusammensetzung nach Anspruch 5, bei der der aromatische Kohlenwasserstoff gewählt ist aus der durch Toluen, die Xylene, Duren, Isoduren, Pentamethylbenzen, a-Methylnaphtalen, 2,6-dimethyl-antracen gebildeten Gruppe.

7. Katalytische Zusammensetzung nach einem der vorhergehenden Ansprüche, bei der das nicht-wässrige Medium ein organisches Derivat des Aluminiums (D) der allgemeinen Formel AlRₓX₃₋ₓ ist, wobei R ein linearer oder verzweigter Alkylrest ist, der 2-8 Kohlenstoffatome umfaßt und X Chlor oder Brom ist und x gleich 1, 2 oder 3 ist.

8. Katalytische Zusammensetzung nach Anspruch 7, bei der das organische Derivat des Aluminiums gewählt ist aus der Gruppe, die gebildet ist aus Dichlorethylaluminium, Dichlorisobutylaluminium, Chlordiethylaluminium, Ethylaluminiumsesquichlorid, Diisobutylaluminiumsesquichlorid.

9. Katalytische Zusammensetzung nach einem der vorhergehenden Ansprüche, bei der das Molverhältnis A:B zwischen 1:0,5 und 1:3 liegt und das Molverhältnis B:C zwischen 1:1 und 1:100 liegt.

10. Katalytische Zusammensetzung nach einem der vorhergehenden Ansprüche, bei der das Molverhältnis A:B bevorzugt zwischen 1:1 und 1:2, das Molverhältnis B:C bevorzugt zwischen 1:1 und 1:10 liegt.

11. Katalytische Zusammensetzung nach einem der Ansprüche 7 bis 10, bei der das Molverhältnis B:D zwischen 1:0 und 1:10 liegt.

12. Katalytische Zusammensetzung nach einem der Ansprüche 7 bis 11, bei der das Molverhältnis B:D bevorzugt zwischen 1:0,01 und 1:5 liegt.

13. Katalytische Zusammensetzung nach einem der vorhergehenden Ansprüche, bei der die Nickelverbindung aus der Gruppe gewählt ist, die gebildet ist durch das Chlorid, das Bromid, das Sulfat, das Acetylacetonat, die Carboxylate und die Phenolate des Nickels.

14. Katalytische Zusammensetzung nach einem der vorhergehenden Ansprüche, bei der das Phosphin gewählt ist aus der Gruppe, die gebildet ist durch Triisopropylphosphin, Tricyclohexylphosphin, Tribenzylphosphin und Tetracyclohexylmethylendiphosphin.

15. Verfahren zur Dimerisierung, Kodimerisierung oder Oligomerisierung wenigstens eines Olefins, Verfahren bei dem das Olefin kontaktiert wird mit wenigstens einer Verbindung des Nickels im Gemisch oder im Komplex mit wenigstens einem tertiären Phosphin, wobei diese Verbindung wenigstens zum Teil in einem nicht-wässrigen Medium ionischen Charakters gelöst ist, dadurch gekennzeichnet,
daß dieses Medium aus der Kontaktierung wenigstens eines Aluminiumhalogenids mit wenigstens einem quarternären Ammoniumhalogenid und/oder wenigstens einem quarternären Phosphoniumhalogenid und mit wenigstens einem aromatischen Kohlenwasserstoff resultiert.

16. Verfahren nach Anspruch 15, bei dem die Reaktionstemperatur mindestens zwischen -40° und +70°C und der Druck zwischen 0,1 und 20 MPa beträgt.

17. Verfahren nach einem der Ansprüche 15 oder 16, bei dem die Reaktionstemperatur mit dem Olefin zwischen -20° und +50°C liegt.

18. Verfahren nach einem der Ansprüche 15 bis 17, bei dem der aromatische Kohlenwasserstoff während der Reaktion mit dem Olefin eingeführt wird.
